# EUROPEAN PATENT APPLICATION

(11) **EP 3 871 512 A1**
(43) Date of publication of application: **01.09.2021**
(21) Application number: 21159326.4
(22) Date of filing: 25.02.2021
(51) Int. Cl.: A23L 2/38, A23L 2/52, A23L 33/105, A61K 36/185, C12C 5/00, C12C 11/00, C12C 12/00, C12C 12/04, C12H 3/00

(54) **PROCESS OF THC EXTRACTION BY FERMENTATION**

(30) Priority: 26.02.2020 US 202016801987
(71) Applicant: Hyatt, Joseph, Nashua, New Hampshire 03062 (US)
(72) Inventor: Hyatt, Joseph, Nashua, New Hampshire 03062 (US)
(74) Representative: AWA Sweden AB

(57) **Abstract**

A process and method of fermenting a wort, juice, tea or base beverage by the addition of a yeast and decarboxylated ground cannabis in order to extract the tetrahydrocannabinol (THC) into a non-alcoholic beverage. This technique involves the steps of: extracting THC through fermentation, limiting the beverages alcoholic potential by stopping the fermentation early, thereby creating a THC infused non- alcoholic beverage that has a composition with a unique ester and phenol profile only found in beverages that have undergone the process of fermentation.

## Description

### PATENTS CITED:

The following documents and references are incorporated by reference in their entirety, Ruben et al (US Pat. No. 9,950,275), Alfiere (US Pat. No. 9,913,868), Lewis et al (US Pat. No. 9,095,554) and Whittle et al (US Pat. No. 7,622,140).

### FIELD OF THE INVENTION

The invention relates in general to a process and method for extracting decarboxylated tetrahydrocannabinol (THC) through fermentation, and specifically to the creation of a cannabis infused non-alcoholic beverage.

### DESCRIPTION OF THE RELATED ART

There are many ways that tetrahydrocannabinol, hereinafter referred to as THC, generally to be found in cannabis may be extracted. Two common prior arts for extraction of THC in the cannabis field are CO2 extraction and butane extraction. These processes were created due to demand from consumers, who are always looking for new ways to consume THC.

Fermentation has been used for thousands of years to the create alcoholic beverages. In general, fermentation is used in the brewery industry, not the cannabis industry. The creation of an alcoholic beverage containing more than .05% alcohol by volume, hereinafter referred to as ABV, is controlled.

However, anything that contains less than .05% ABV is considered a non-alcoholic beverage. Through fermentation, while creating a non-alcoholic fermented beverage, an individual can extract THC into the beverage without crossing the ABV threshold that would consider this an alcoholic beverage. Such a process would allow for the non-alcoholic beverage to acquire unique esters and phenols only achieved through fermentation while also extracting the THC for consumption.

### SUMMARY OF THE INVENTION

This section is for the purpose of summarizing some aspects of the present invention and to briefly introduce some preferred embodiments. Simplifications or omissions may be made to avoid obscuring the purpose of the section. Such simplifications or omissions are not intended to limit the scope of the present invention.

In one aspect the invention is about a method for fermenting a beverage, said method comprising the steps of starting a fluid base made from a wort, juice, tea or base beverage, stabilizing said fluid base temperature at between 7 and 38 deg. C, adding one or more yeasts and/or cultures, adding decarboxylated cannabis, allowing the fermentation of the combined fluid until said combined fluid Alcohol by volume (ABV) is above 0.02% but below 0.049% and lowering said combined fluid to below 3 deg C. In another aspect its about filtering said combined fluid through a mesh with openings no greater than 300 microns. In yet another aspect about bottling or kegging said combined fluid. In another aspect, said yeast is one selected from a group comprised by at least one of: Brettanomyces yeast and or lactobacillus cultures, ale yeast, Saccharomyces cerevisiae, lager yeast, Saccharomyces pastorianus and/or a S.C.O.B.Y. (Symbiotic Culture of Bacteria and Yeast).

In one aspect, the invention is about a beverage prepared by a method comprising forming a combined fluid comprised of one or more sugars and one or more fluids, adding to said combined fluid one or more yeasts and/or cultures, adding decarboxylated cannabis, stabilizing said fluid base temperature at between 7 and 38 deg. C, allowing the fermentation of the combined fluid until said combined fluid ABV is above 0.02% but below 0.049% and lowering said combined fluid to below 3 deg C. In another aspect it's about filtering said combined fluid through a mesh with openings no greater than 300 microns. In yet another aspect about bottling or kegging said combined fluid. In another aspect, said yeast is one selected from a group comprised by at least one of: Brettanomyces yeast and or lactobacillus cultures, ale yeast, Saccharomyces cerevisiae, lager yeast, Saccharomyces pastorianus and/or a S.C.O.B.Y. (Symbiotic Culture of Bacteria and Yeast).

Other features and advantages of the present invention will become apparent upon examining the following detailed description of an embodiment thereof, taken in conjunction with the attached drawings.

### BRIEF DESCRIPTION OF THE DRAWINGS

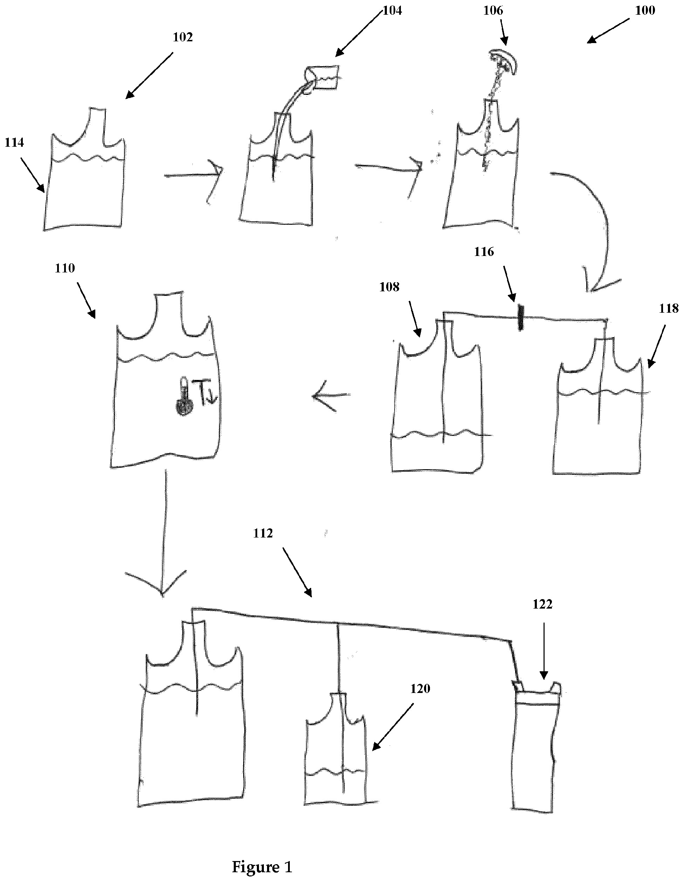

**FIG.** 1 shows an illustration of a THC extracting process, according to an exemplary embodiment of the invention.

The above-described and other features will be appreciated and understood by those skilled in the art from the following detailed description, drawings, and appended claims.

### DETAILED DESCRIPTION OF THE PREFERRED EMBODIMENT

This section is for the purpose of summarizing some aspects of the present invention and to briefly introduce some preferred embodiments. Simplifications or omissions may be made to avoid obscuring the purpose of the section. Such simplifications or omissions are not intended to limit the scope of the present invention.

To provide an overall understanding of the invention, certain illustrative embodiments and examples will now be described. However, it will be understood by one of ordinary skill in the art that the same or equivalent functions and sequences may be accomplished by different embodiments that are also intended to be encompassed within the spirit and scope of the disclosure. The compositions, apparatuses, systems and/or methods described herein may be adapted and modified as is appropriate for the application being addressed and that those described herein may be employed in other suitable applications, and that such other additions and modifications will not depart from the scope hereof.

Simplifications or omissions may be made to avoid obscuring the purpose of the section. Such simplifications or omissions are not intended to limit the scope of the present invention. All references, including any patents or patent applications cited in this specification are hereby incorporated by reference. No admission is made that any reference constitutes prior art. The discussion of the references states what their authors assert, and the applicants reserve the right to challenge the accuracy and pertinence of the cited documents. It will be clearly understood that, although a number of prior art publications are referred to herein, this reference does not constitute an admission that any of these documents form part of the common general knowledge in the art.

As used in the specification and claims, the singular forms "a", "an" and "the" include plural references unless the context clearly dictates otherwise. For example, the term "a transaction" may include a plurality of transaction unless the context clearly dictates otherwise. As used in the specification and claims, singular names or types referenced include variations within the family of said name unless the context clearly dictates otherwise.

Certain terminology is used in the following description for convenience only and is not limiting. The words "lower," "upper," "bottom," "top," "front," "back," "left," "right" and "sides" designate directions in the drawings to which reference is made, but are not limiting with respect to the orientation in which the modules or any assembly of them may be used.

It is acknowledged that the term 'comprise' may, under varying jurisdictions, be attributed with either an exclusive or an inclusive meaning. For the purpose of this specification, and unless otherwise noted, the term 'comprise' shall have an inclusive meaning-i.e. that it will be taken to mean an inclusion of not only the listed components it directly references, but also other non-specified components or elements. This rationale will also be used when the term 'comprised' or 'comprising' is used in relation to one or more steps in a method or process.

Referring to **FIG. 1** we see a proposed embodiment of the process **100,** which we begin by through the fermentation of any appropriate wort, juice, tea or base beverage **102** within an appropriate container **114.** The fluid to be fermented may be based on any traditional wort (such as those used for beer, i.e. grains, hops, etc., as long as the sugars traditionally required for fermentation to begin are present). The fermentation is started after adding **104** one or more types of yeast. In one embodiment, this may be a wild yeast such as one of: Brettanomyces yeast and or lactobacillus cultures, ale yeast, Saccharomyces cerevisiae, lager yeast, Saccharomyces pastorianus and/or a S.C.O.B.Y. (Symbiotic Culture of Bacteria and Yeast).

To ferment efficiently, the temperature of the mix is kept between 7 and 38 degrees C (45 to 100 degrees F). When starting the fermentation, we add **106** any strain of decarboxylated ground cannabis into the fermenting beverage. In this fashion, the THC is extracted from the ground cannabis into the fluid through the fermentation process, accomplishing the mixing of their properties seamlessly.

The process of decarboxylating involves applying low heat for an extended period of time to convert cannabis' non-intoxicating tetrahydrocannabinolic acid (THCA) into THC, the cannabinoid known for its psychoactive effects. Without decarboxylation, the effects of DIY edibles may be weak or lackluster, at best.

In one embodiment, the beverage is allowed to ferment to under .049% ABV (typically in one to five days **108** or any other suitable alcohol level), at which point the fermentation is stopped. By doing this, the appropriate flavors and traces of a beer or similar alcoholic beverage are generated. Then, by first filtering the fluid through a particle filter (preferably one having a screen **116** allowing only particles of a size of 300 microns or less) into a clean container **118**, then dropping the fluid temperature **110** in said new container to about 3 deg C (∼37 degrees F) or below (preferably avoiding freezing, in one embodiment).

Once the beverage has settled and fully stopped fermentation **112,** it is filtered and placed into either bottles **122** or kegs **124.** Said bottled product creates a unique non-alcoholic THC beverage that contain esters and phenols only found in fermentation. This is critical, since it provides the 'traditional' beer taste queues, without the alcohol.

### CONCLUSION

In concluding the detailed description, it should be noted that it would be obvious to those skilled in the art that many variations and modifications can be made to the preferred embodiment without substantially departing from the principles of the present invention. Also, such variations and modifications are intended to be included herein within the scope of the present invention as set forth in the appended claims. Further, in the claims hereafter, the structures, materials, acts and equivalents of all means or step-plus function elements are intended to include any structure, materials or acts for performing their cited functions.

It should be emphasized that the above-described embodiments of the present invention, particularly any "preferred embodiments" are merely possible examples of the implementations, merely set forth for a clear understanding of the principles of the invention. Any variations and modifications may be made to the above-described embodiments of the invention without departing substantially from the spirit of the principles of the invention. All such modifications and variations are intended to be included herein within the scope of the disclosure and present invention and protected by the following claims.

The present invention has been described in sufficient detail with a certain degree of particularity. The utilities thereof are appreciated by those skilled in the art. It is understood to those skilled in the art that the present disclosure of embodiments has been made by way of examples only and that numerous changes in the arrangement and combination of parts may be resorted without departing from the spirit and scope of the invention as claimed. Accordingly, the scope of the present invention is defined by the appended claims rather than the foregoing description of embodiments.

## Claims

1. A method for fermenting a beverage, said method comprising the steps of;
starting a fluid base made from a wort, juice, tea or base beverage; stabilizing said fluid base temperature at between 7 and 38 deg. C;
adding one or more yeasts and/or cultures;
adding decarboxylated cannabis;
allowing the fermentation of the combined fluid until said combined fluid Alcohol by volume (ABV) is above 0.02% but below 0.049%; and
lowering said combined fluid to below 3 deg C.

2. the method of claim **1** further comprising;
filtering said combined fluid through a mesh with openings no greater than 300 microns.

3. the method of claim **2** further comprising;
bottling or kegging said combined fluid.

4. the method of claim **1** wherein;
said yeast is one selected from a group comprised by at least one of:
Brettanomyces yeast and or lactobacillus cultures, ale yeast, Saccharomyces cerevisiae, lager yeast, Saccharomyces pastorianus and/or a S.C.O.B.Y. (Symbiotic Culture of Bacteria and Yeast).

5. the method of claim **4** further comprising:
filtering said combined fluid through a mesh with openings no greater than 300 microns.

6. the method of claim **5** further comprising;
bottling or kegging said combined fluid.

7. A beverage prepared by a method comprising:
forming a combined fluid comprised of one or more sugars and one or more fluids:
adding to said combined fluid one or more yeasts and/or cultures;
adding decarboxylated cannabis;
stabilizing said fluid base temperature at between 7 and 38 deg. C;
allowing the fermentation of the combined fluid until said combined fluid ABV is above 0.02% but below 0.049%; and
lowering said combined fluid to below 3 deg C.

8. the beverage of claim **7** further comprising;
filtering said combined fluid through a mesh with openings no greater than 300 microns.

9. the beverage of claim **8** further comprising;
bottling or kegging said combined fluid.

10. the beverage of claim **9** wherein;
said yeast is one selected from a group comprised by at least one of:
Brettanomyces yeast and or lactobacillus cultures, ale yeast, Saccharomyces cerevisiae, lager yeast, Saccharomyces pastorianus and/or a S.C.O.B.Y. (Symbiotic Culture of Bacteria and Yeast).
